# EUROPEAN PATENT SPECIFICATION

(11) **EP 1 404 866 B1**
(45) Date of publication and mention of the grant of the patent: **03.09.2008**
(21) Application number: 02763129.0
(22) Date of filing: 10.07.2002
(51) Int. Cl.: C12Q 1/66, C12Q 1/68

(54) **BIOASSAY FOR MYOSTATIN**
BIOASSAY FÜR MYOSTATIN
TITRAGE BIOLOGIQUE DE MYOSTATINE

(30) Priority: 11.07.2001 NZ 51286901
(43) Date of publication of application: 07.04.2004
(73) Proprietor: ORICO Limited, Dunedin (NZ)
(72) Inventor: KAMBADUR, Ravi, 2001 Hamilton (NZ); SHARMA, Mridula, 2001 Hamilton (NZ); LANGLEY, Brett, 2001 Hamilton (NZ)
(74) Representative: Andrews, Timothy Stephen
(86) International application number: PCT/NZ2002/000120
(87) International publication number: WO 2003/006686

(56) References cited:
- EP-A1- 1 072 680
- WO-A-00/43781
- WO-A1-00/01810
- WO-A2-00/04051
- WO-A2-00/77206
- KAMBADUR ET AL: "Mutations in myostatin (GDF8) in double-muscled Belgian Blue and Piedmontese cattle" GENOME RESEARCH, COLD SPRING HARBOR LABORATORY PRESS, US, vol. 7, no. 9, 1 September 1997 (1997-09-01), pages 910-916, XP002085802 ISSN: 1088-9051

## Description

### Technical Field

The present invention relates to an *in vitro* bioassay for myostatin. In particular, the invention broadly concerns an assay for biologically active myostatin.

### Background to the Invention

*Myostatin* (Growth and Differentiation Factor -8) was cloned from mouse and was shown to be a negative regulator of muscle growth [1]. Myostatin expression is restricted to the myotome compartment of developing somites, and is also expressed in various skeletal muscles in the adult animal. Myostatin knock-out mice exhibit a highly increased skeletal muscle mass. The increase in skeletal muscle mass appears to be widespread throughout the body, and isolated muscles from myostatin null mice weigh about 2-3 times more than wild type muscles. The massive skeletal muscle enlargement observed in the knock-out mice however, is not merely due to an increase in muscle fibre number (hyperplasia) but also due to muscle fibre thickness (hypertrophy). The cross-sectional muscle area of the myostatin knock-out mice is increased by about 14 to 49% depending on muscle type. Further, myostatin null mice have been shown to be viable and fertile.

In 1997 the inventors discovered that two breeds of cattle Belgian Blue and Piedmontese, both characterised by increased muscle mass, have mutations in the myostatin coding sequence [2]. This phenomenon of "double muscling" has been observed in many breeds of cattle for the past 190 years, and on average the animals have 20-25% increase in muscle mass.

Although expression of myostatin is mainly restricted to skeletal muscle there is a low level expression in adipose tissue and heart muscle. The physiological role of myostatin in the adult individual is not known, although it appears that myostatin may function as a specific negative regulator of skeletal muscle growth. Myostatin may be implicated in exercise induced muscle hypertrophy and regeneration after muscle injury. Myostatin may also suppress adipose tissue growth. It is also not known whether myostatin works locally or systemically during the postnatal growth of the animal.

The Myostatin protein is synthesized as a precursor and then proteolytically processed to give active C-terminal processed (mature) myostatin and an N-terminal Latency Associated Peptide (LAP). Based on primary structure similarities to other TGF-β members, once processed, myostatin is secreted into circulation [3]. The circulating processed myostatin complex, possibly binds to activin type II β receptors to initiate downstream signaling events. Thus, assessing the levels of the active myostatin protein complex in circulation would be useful as a diagnostic tool to analyse muscle physiology. Particularly given it has been recently shown that in the muscle wasting conditions associated with HIV infection the circulatory levels of myostatin increase [3].

The current methods used to detect myostatin in circulation to date, such as Radioimmuno Assay (RIA) and ELISA, can only detect the total levels of myostatin and do not differentiate between the biologically active and inactive forms of myostatin.

It would therefore be useful if there were a research tool which could assist in discovery of the physiological role of myostatin in animals.

Accordingly, the present invention is broadly directed to a myostatin promoter and reporter gene bioassay and its uses in determining the levels of biologically active myostatin present within an animal, or *in vitro* cell or tissue cultures.

WO 00/43781 discloses a bioassay for measuring myostatin (GDF-8) inhibitory activity, in which myosarcoma cells transfected with a functional variant of the myostatin promoter controlling expression of a reporter gene are exposed to myostatin, whereby myostatin induces an increase in reporter gene expression.

It is an object of the present invention to address the foregoing problems or at least to provide the public with a useful choice.

Further aspects and advantages of the present invention will become apparent from the ensuing description which is given by way of example only.

### Disclosure of Invention

According to a first aspect of the present invention there is provided an *in vitro* bioassay for determining the amount of active myostatin present in an animal or part thereof comprising of steps:
a) amplifying the myostatin promoter, or a functional fragment or variant thereof that has promoter activity which is down-regulated by myostatin;
b) cloning the promoter, or a functional fragment or variant thereof that has promoter activity which is down-regulated by myostatin, into a reporter vector or construct having a reporter gene
c) transfecting the vector or construct into myoblasts and selecting transfected myoblasts;
d) incubating transfected myoblasts with a myostatin, serum or muscle extract taken from an animal of interest whereby expression of a reporter gene is reduced;
e) washing the myoblasts and preparing protein extracts therefrom;
f) performing the reporter gene assay on the protein extracts for determining expression of the reporter gene.

For the purposes of the specification it will be clearly understood that the word "comprising" means "including but not limited to," and that the word "comprises" has a corresponding meaning.

It is envisaged that the present invention may have application to all animals which utilise myostatin as a growth regulating factor.

In particular the present invention may have application to mammals or other vertebrates.

Preferably, said mammal may be selected from the group consisting of: humans, sheep, cattle, goats, deer, horses, camels, possum, pigs, mice, rats; and said vertebrates may be selected from the group consisting of: birds, chickens, fish, crocodiles and alligators. Other mammals or vertebrates of commercial importance which utilise myostatin to regulate muscle growth are also envisaged.

The promoter region may comprise the entire myostatin promoter sequence, said sequence being the same as described in WO/00/01810, as herein listed in SEQ ID NO.1. Preferably however, the promoter region to be amplified may comprise the 1.6 kb region of the myostatin promoter immediately upstream of the myostatin gene or a functional fragment or variant of said region that has promoter activity which is down-regulated by myostatin.

The promoter may be mammalian or vertebrate and is preferably selected on the basis of the type of animal to be tested.

The promoter molecule may be an RNA, cRNA, genomic DNA or cDNA molecule, and may be single- or doublestranded. The promoter molecule may also optionally comprise one or more synthetic, non-natural or altered nucleotide bases, or combinations thereof.

In preferred embodiments, the amplification step may be performed by any convenient method, such as the polymerase chain reaction, or ligase chain reaction. However, other amplification techniques may be employed without departing from the scope of the present invention.

The cloning of the promoter into the vector may be achieved by any suitable cloning method. Preferably, the cloning method may be selected from any disclosed in Sambrook et al [6].

A "cloning vector" refers to a nucleic acid molecule originating or derived from a virus, a plasmid or a cell of a higher organism into which another exogenous (foreign) nucleic acid molecule of interest, of appropriate size can be integrated without loss of the vector's capacity for self-replication. Thus vectors can be used to introduce at least one foreign nucleic acid molecule of interest (e.g. gene of interest) into host cells, where the gene can be reproduced in large quantities.

The cloning vector may be selected according to the myoblasts to be used in the assay. Useful vectors will generally have the following characteristics:
(a) the ability to self-replicate;
(b) the possession of a single target for any particular restriction endonuclease; and
(c) desirably, carry genes for a readily selectable marker such as antibiotic resistance. Preferably, the reporter gene is luciferase.

Two major types of vector possessing these characteristics are plasmids and bacterial viruses (bacteriophages or phages). Presently preferred vectors may include the following: the pUC, pBlueScript, pGEM, PGEX, pBK-CMV, lambda ZAP, lambda GEM and pSP series. However, this list should not be seen as limiting the scope of the present invention.

To further ensure the expression of the reporter gene, via the myostatin promoter, the reporter vector may also include additional control sequences, such as origins of replication, enhancer and transcriptional terminator sequences all operably linked to the reporter gene. The selection of the additional control sequences to be included in the reporter vector may vary and will be dependent on the type of myoblasts intended to be used in the assay.

The vector or construct may be introduced into myoblasts according to standard techniques. For example, the calcium phosphate precipitation method of Graeme *et al*, 1978 [7] is preferred, however other techniques may also be employed without departing from the scope of the present invention.

The term "introducing" (or grammatical variants thereof) when used in the context of inserting a nucleic acid molecule into a cell, means "transfection" or "transformation" or "transduction" and includes reference to the incorporation or transfer of a nucleic acid molecule into a eukaryotic or prokaryotic cell where the nucleic acid molecule may be incorporated into the genome of the cell (e.g. chromosome, plasmid, plastid, mitochondrial DNA), converted into an autonomous replicon, or transiently expressed (e.g. transfected mRNA).

The term "transfection" as used herein, refers to the uptake, incorporation, and expression of recombinant DNA by eukaryotic cells.

The term "transformation" as used herein refers to a process by which the genetic material carried by an individual cell is altered by incorporation of exogenous DNA into its genome.

The term "transduction" as used herein, refers to the process of transferring genetic information from a nucleic acid molecule from one cell to another by way of a viral vector.

In general any myoblasts may be used for transfection with the vector. Most preferably however, the myoblasts are C2C12 muscle precursor cells. Similarly, any assay may be used which is capable of the reporter gene being utilised in either transiently or stably transfected cells.

### Brief Description of Drawings

Further aspects of the present invention will become apparent from the following non-limiting description which is given by way of example only and with reference to the accompanying drawings and example(s) in which:
- Figure 1: is a graph of Luciferase activity illustrating that the myostatin promoter is capable of driving expression of luciferase.
- Figure 2: is a graph of Luciferase activity showing that myoblasts expressing luciferase, under the control of the myostatin promoter, reduce expression of luciferase when exposed to increasing concentrations of myostatin protein.

### Best Modes for Performing the Invention

Non-limiting examples illustrating the invention will now be provided. It will be appreciated that the above description is provided by way of example only and variations in both the materials and techniques used which are known to those persons skilled in the art are contemplated.

Recently, the inventors identified and cloned the myostatin promoter and have described this in WO 00/01810 (herein listed in SEQ ID NO. 1). In particular, the inventors have discovered an approximately 1.6 kb region of this promoter, immediately upstream of the myostatin gene, which is able to drive the expression of a reporter gene.

Given, genetic models such as Belgian Blue cattle, which express inactive myostatin, have higher levels of mutated (non-functional) myostatin mRNA [4], this suggests that myostatin regulates its own expression via a negative feedback mechanism.

Utilising the 1.6kb myostatin promoter region and a suitable reporter gene the inventors have now managed to confirm that exogenously added myostatin can indeed down regulate myostatin promoter activity in a dose dependent manner.

### Examples

### Example 1

### Construction of the reporter plasmid and selection of the stable cell lines

The genetic evidence suggested that myostatin regulates its own promoter by a " feed back inhibition" loop. We thus, wanted to study if the necessary regulatory elements for the feedback inhibition are localized within the 1.6 kb of myostatin promoter. For this purpose, the 1.6 kb bovine myostatin promoter was PCR amplified using RK55 and RK56 primers and cloned into TA cloning vector pGEM-T easy. The promoter was then mobilised into the reporter plasmid pGL3-basic as a *KpnI* fragment. To generate a stable cell line that harbours the myostatin promoter-reporter system, the myostatin promoter and luciferase reporter gene were subsequently mobilised as a *Not I* and *Xho I* fragment into pcDNA3 and 12.5 µg was transfected into C2C12 myoblasts cultured in DMEM using Lipofectamine 2000 according to the manufacturer's protocol. Stably transfected C2C12 cells were selected for their resistance to geneticin (40µg/ml).

### Dose response of myostatin promoter to myostatin protein

To determine the sensitivity of the myostatin promoter inhibition by the myostatin protein, stably transfected C2C12 cells were incubated with increasing concentration of myostatin protein (0.5 to 4 µg/ml) for 24 hours in DMEM media with 10% FCS and cell extracts were made from the myoblasts to determine the luciferase activity.

### Luciferase assay

Cells to be analysed for luciferase assay were lysed in 300 µl of 1 x Reporter lysis buffer (Promega). Lysates were collected and vortexed for 10 sec. After a quick freeze-thaw the lysates were centrifuged at 12,000xg for 15 sec and 10 µl supernatants were analysed for luciferase reporter gene activity (Promega) in a Turner Designs Luminometer (Model TD-20/20). The total proteins were estimated by Bradford Bio-Rad protein assay reagent (Bio-Rad Lab).

### Results

### Myostatin promoter activity

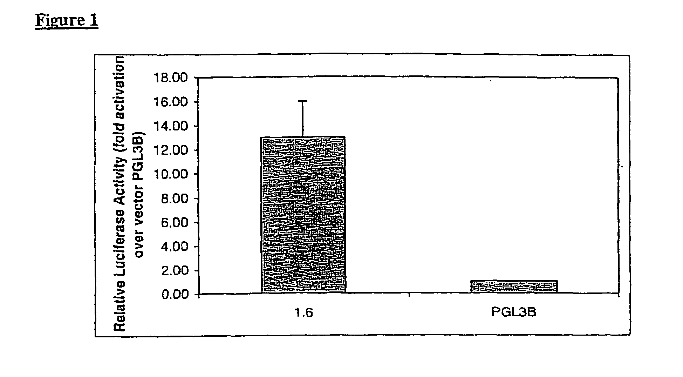

To determine the regions within the bovine 5' *myostatin* genomic DNA that might specify functional promoter activity, 1.6 kb of myostatin upstream genomic DNA was sub-cloned into pGL3-basic vector and transfected into C2C12 cells. The reporter gene activity was measured by luciferase assay. As shown in Fig. 1, DNA fragment that initiated at the 5' end at -1.6 kb and terminated at +43 displayed significant reporter gene activity relative to the control vector in C2C12 cells.

### Myostatin inhibits its own promoter by feedback inhibition

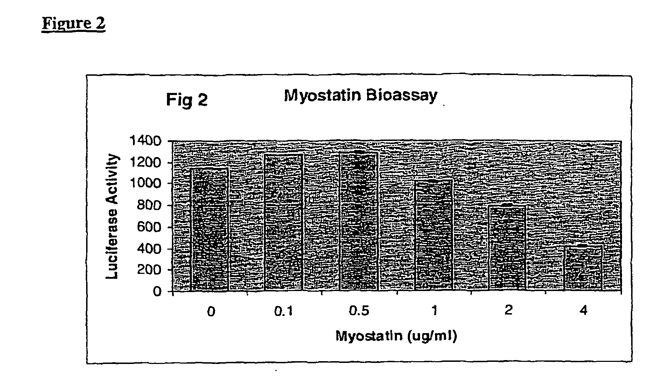

Myostatin gene expression analysis done in normal muscled and double-muscled animals (that lack functional myostatin) seem to suggest that myostatin inhibits its own expression by negative feedback mechanism. To investigate if the 1.6 kb myostatin promoter has the necessary elements for feed back inhibition, the 1.6 kb promoter-luciferase reporter portion in pGL3-basic was subcloned into pcDNA3, transfected into C2C12 cells and selected for stable genomic integration. Cells harbouring the myostatin promoter and reporter system were incubated with increasing concentrations of recombinant processed myostatin and the luciferase activity was measured. As shown in Fig 2, increasing concentration of myostatin decreased the luciferase activity. The assay appears to be sensitive since a change of 0.5 µg/ml resulted in significant decrease in luciferase activity.

### Discussion

Myostatin is a member of TGF beta superfamily. Mutations in myostatin lead to hyperplasia and hypertophy of skeletal muscle. Here we have developed a bioassay using the myostatin promoter region involved in feedback inhibition caused by the myostatin protein. Using luciferase reporter system we first showed that 1.6 kb of myostatin upstream region is sufficient to drive the expression of luciferase reporter gene in myoblasts (Fig 1). When C2C12 myoblasts harbouring the reporter construct were incubated with increasing concentrations of myostatin, the promoter activity significantly decreased in a dose dependent manner. This indicates that the necessary regulatory elements that inhibit myostatin transcription by feedback inhibition are located within 1.6 kb. Furthermore, the auto-inhibition appears to be very sensitive and dependent on myostatin protein levels. Since there is an inverse relationship with the exogenous myostatin levels and luciferase reporter activity we have standardised a bioassay for myostatin using myostatin promoter reporter system. The possible mechanism that regulates the feedback inhibition of myostatin could be through one or more of the cis-regulatory elements present within 1.6 kb. The 1.6 kb of myostatin promoter region contains at least 10 E box motifs which are the binding sites for the myogenic regulatory factors such as MyoD and Myf-5 [5].

### References

- 1.: McPherron, A.C., A.M. Lawler, and S.J. Lee, Regulation of skeletal muscle mass in mice by a new TGF-beta superfamily member. Nature, 1997. 387(6628): p. 83-90.
- 2.: Kambadur, R., et al., Mutations in myostatin (GDF8) in double-muscled Belgian Blue and Piedmontese cattle. Genome Res, 1997. 7(9): p. 910-6.
- 3.: Gonzalez-Cadavid, N.F., et al., Organization of the human myostatin gene and expression in healthy men and HIV-infected men with muscle wasting. Proc Natl Acad Sci U S A, 1998. 95(25): p. 14938-43.
- 4.: Oldham, J.M., et al., Molecular expression of myostatin and MyoD is greater in double-muscled than normal-muscled cattle fetuses. Am. J. Physiol. Regul. Integr. Comp. Physiol., 2001. 280(5): p. 1488-1493.
- 5.: Rudnicki, M.A. and R. Jaenisch, The MyoD family of transcription factors and skeletal myogenesis. Bioessays, 1995. 17(3): p. 203-9.
- 6.: Sambrook, J, Fritsch, E.F. and T. Maniatis, Molecular Cloning: A laboratory manual, 1989. CSHL press. NY, New York, USA.
- 7.: Graeme and Van Der Eb, Virology 52:546 (1978)

## Claims

1. An *in vitro* bioassay for determining the amount of active myostatin present in an animal or part thereof comprising of steps:
a) amplifying the myostatin promoter, or a functional fragment or variant thereof that has promoter activity which is down-regulated by myostatin;
b) cloning the promoter, or a functional fragment or variant thereof that has promoter activity which is down-regulated by myostatin, into a reporter vector or construct having a reporter gene
c) transfecting the vector or construct into myoblasts and selecting transfected myoblasts;
d) incubating transfected myoblasts with a myostatin, serum or muscle extract taken from an animal of interest whereby expression of a reporter gene is reduced;
e) washing the myoblasts and preparing protein extracts therefrom;
f) performing the reporter gene assay on the protein extracts for determining expression of the reporter gene.

2. A method as claimed in claim 1 wherein the promoter region comprises the entire myostatin promoter sequence, said sequence being the same as that listed in SEQ ID. NO.1.

3. A method as claimed in claim 1 wherein the promoter region to be amplified comprises the 1.6 kb region of the myostatin promoter immediately upstream of the myostatin gene, or a functional fragment or variant of said region that has promoter activity which is down-regulated by myostatin.

4. A method as claimed in any one of the preceding claims wherein the promoter is mammalian or vertebrate and is selected on the basis of the type of animal to be tested.

5. A method as claimed in claim 4 wherein said animal is a mammal selected from the group consisting of: humans, sheep, cattle, goats, deer, horses, camels, possum, pigs, mice and rats.

6. A method as claimed in claim 4 wherein said animal is a vertebrate selected from the group consisting of: birds, chickens, fish, crocodiles and alligatos.

7. A method as claimed in claim 1 wherein the myoblasts are C2C12 muscle precursor cells.

## Patentansprüche

1. *In vitro*-Bioassay für die Bestimmung der in einem Tier oder einem Teil davon vorhandenen Menge von aktivem Myostatin, umfassend die Schritte:
(a) Amplifizieren des Myostatin-Promotors oder eines funktionellen Fragments oder einer Variante davon, die über Promotoraktivität verfügt, die durch Myostatin herabreguliert wird;
(b) Klonieren des Promotors oder eines funktionellen Fragments oder einer Variante davon, die über Promotoraktivität verfügt, die durch Myostatin herabreguliert wird, in einen Reportervektor oder Konstrukt, das über ein Reportergen verfügt;
(c) Transfektieren des Vektors oder Konstrukts in Myoblasten und Selektieren transfektierter Myoblasten;
(d) Inkubieren transfektierter Myoblasten mit einem Myostatin, Serum oder Muskelextrakt, die von einem Tier, das von Interesse ist, genommen wurden, wodurch die Expression eines Reportergens reduziert wird;
(e) Waschen der Myoblasten und Herstellen von Proteinextrakten daraus;
(f) Ausführen des Reportergen-Assays auf den Proteinextrakten zur Bestimmung der Expression des Reportergens.

2. Verfahren nach Anspruch 1, wobei das Reportergen die gesamte Myostatin-Promotorsequenz aufweist und diese Sequenz dieselbe ist wie die in SEQ.ID.No.1 aufgeführte Sequenz.

3. Verfahren nach Anspruch 1, wobei die Promotorregion, die amplifiziert werden soll, die 1,6kb-Region des Myostatin-Promotors unmittelbar stromaufwärts des Myostatin-Gens aufweist oder ein funktionelles Fragment oder eine Variante dieser Region, die über Promotoraktivität verfügt, die durch Myostatin herabreguliert wird.

4. Verfahren nach einem der vorgenannten Ansprüche, wobei der Promotor von Säugern stammt oder von Vertebraten und ausgewählt ist auf der Grundlage des Typs des Tieres, das getestet werden soll.

5. Verfahren nach Anspruch 4, wobei das Tier ein Säuger ist, ausgewählt aus der Gruppe, bestehend aus: Menschen, Schaf, Rind, Ziegen, Hirsche, Pferden, Kamelen, Opossum, Schweinen, Mäusen und Ratten.

6. Verfahren nach Anspruch 4, wobei das Tier ein Vertebrat ist, ausgewählt aus der Gruppe, bestehend aus: Vögeln, Hühnern, Fisch, Krokodilen und Alligatoren.

7. Verfahren nach Anspruch 1, wobei die Myoblasten C2C12-Muskel-Präkursorzellen sind.

## Revendications

1. Essai biologique *in vitro* pour la détermination de la quantité de myostatine active présente dans un animal ou une partie de celui-ci comprenant les étapes :
a) d'amplification du promoteur de la myostatine, ou d'un fragment fonctionnel ou d'un variant de celui-ci qui possède une activité de promoteur qui est régulée à la baisse par la myostatine ;
b) le clonage du promoteur, ou d'un fragment fonctionnel ou d'un variant de celui-ci qui possède une activité de promoteur qui est régulée à la baisse par la myostatine, dans un vecteur rapporteur ou une construction ayant un gène rapporteur ;
c) la transfection du vecteur ou de la construction dans des myoblastes et la sélection des myoblastes transfectés ;
d) l'incubation des myoblastes transfectés avec une myostatine, du sérum ou un extrait musculaire prélevé à partir d'un animal d'intérêt moyennant quoi l'expression d'un gène rapporteur est réduite ;
e) le lavage des myoblastes et la préparation des extraits protéiques de ceux-ci ;
f) la réalisation de l'essai de gène rapporteur sur les extraits protéiques pour la détermination de l'expression du gène rapporteur.

2. Procédé tel que revendiqué selon la revendication 1, dans lequel la région promotrice comprend la séquence entière du promoteur de la myostatine, ladite séquence étant identique à celle listée dans SEQ ID N° 1.

3. Procédé tel que revendiqué selon la revendication 1, dans lequel la région promotrice à amplifier comprend la région de 1,6 kb du promoteur de la myostatine immédiatement en amont du gène de la myostatine, ou un fragment fonctionnel ou un variant de ladite région qui possède une activité de promoteur qui est régulée à la baisse par la myostatine.

4. Procédé tel que revendiqué selon l'une quelconque des revendications précédentes, dans lequel le promoteur est un mammifère ou un vertébré et est choisi sur la base du type d'animal à tester.

5. Procédé tel que revendiqué selon la revendication 4, dans lequel ledit animal est un mammifère choisi parmi le groupe constitué : des êtres humains, du mouton, du boeuf, des chèvres, des cerfs, des chevaux, des chameaux, de l'opossum, des porcs, des souris et des rats.

6. Procédé tel que revendiqué selon la revendication 4, dans lequel ledit animal est un vertébré choisi parmi le groupe constitué : des oiseaux, des poulets, du poisson, des crocodiles et des alligators.

7. Procédé tel que revendiqué selon la revendication 1, dans lequel les myoblastes sont des cellules C2C12 précurseurs du muscle.
